(19) **Europäisches Patentamt / European Patent Office / Office européen des brevets**

(11) **EP 1 744 789 B1**

(12) **FASCICULE DE BREVET EUROPEEN**

(45) Date de publication et mention
de la délivrance du brevet:
**06.08.2008 Bulletin 2008/32**

(51) Int Cl.:
**A61K 51/12** (2006.01)    **A61K 9/14** (2006.01)
**A61P 35/04** (2006.01)

(21) Numéro de dépôt: **05769516.5**

(22) Date de dépôt: **09.05.2005**

(86) Numéro de dépôt international:
**PCT/FR2005/001145**

(87) Numéro de publication internationale:
**WO 2005/120590 (22.12.2005 Gazette 2005/51)**

(54) **PARTICULES ACTIVABLES PAR DES RAYONS X ET/OU UV, LEUR PREPARATION ET LEUR UTILISATION THÉRAPEUTIQUE OU DIAGNOSTIQUE**

DURCH RÖNTGEN- UND/ODER UV-STRAHLEN AKTIVIERBARE PARTIKEL, DEREN HERSTELLUNG UND DEREN THERAPEUTISCHE ODER DIAGNOSTISCHE VERWENDUNG

X-RAY AND/OR UV ACTIVATABLE PARTICLES, THEIR PREPARATION AND THEIR THERAPEUTIC OR DIAGNOSTIC USES

(84) Etats contractants désignés:
**AT BE BG CH CY CZ DE DK EE ES FI FR GB GR HU IE IS IT LI LT LU MC NL PL PT RO SE SI SK TR**
Etats d'extension désignés:
**AL BA HR LV MK YU**

(30) Priorité: **10.05.2004 FR 0405036**

(43) Date de publication de la demande:
**24.01.2007 Bulletin 2007/04**

(73) Titulaire: **Nanobiotix**
**75012 Paris (FR)**

(72) Inventeurs:
• **LEVY, Laurent**
  **F-75012 Paris (FR)**
• **HOCHEPIED, Jean-François**
  **F-75012 Paris (FR)**
• **BALENCIE, Jérémy**
  **F-77250 Veneux-les-Sablons (FR)**
• **PRASAD, Paras, Nath**
  **Williamsville, NY 14138 (US)**
• **BERGEY, Earl, Jim**
  **South Dayton, NY 14138 (US)**
• **PANAK, Edouard, André**
  **F-31100 Toulouse (FR)**
• **BOUSSAHA, Abdel, Kader**
  **F-75002 Paris (FR)**

(74) Mandataire: **Chajmowicz, Marion et al**
**Cabinet BECKER & Associés**
**25 rue Louis Legrand**
**75002 Paris (FR)**

(56) Documents cités:
**EP-A- 1 162 626**

• **SHIBATA HITOSHI ET AL: "Hydroxyl radical generation depending on O2 or H2O by a photocatalyzed reaction in an aqueous suspension of titanium dioxide" BIOSCIENCE BIOTECHNOLOGY AND BIOCHEMISTRY, vol. 62, no. 12, décembre 1998 (1998-12), pages 2306-2311, XP009042440 ISSN: 0916-8451 cité dans la demande**

Remarques:
Le dossier contient des informations techniques présentées postérieurement au dépôt de la demande et ne figurant pas dans le présent fascicule.

**Description**

La présente demande concerne de nouvelles particules activables utilisables dans le domaine de la santé. Elle concerne notamment des particules composites capables de générer des radicaux libres ou de la chaleur sous excitation aux rayons X, et leurs utilisations en santé, notamment humaine. Les particules de l'invention comprennent un noyau à base de composés inorganiques et, éventuellement, organiques, et elles peuvent être activées in vivo, par excitation externe contrôlable, pour marquer ou altérer des cellules, tissus ou organes. L'invention concerne également des méthodes de production de telles particules, ainsi que des compositions pharmaceutiques ou diagnostiques les contenant.

La thérapie photo dynamique (PDT) a été qualifiée et est maintenant utilisée pour traiter les cancers superficiels comme celui de la peau ou de l'oesophage (voir par exemple McCaughan, J.S. Jr., Drugs and Aging. 15: 49-68 (1999) "Photodynamic Therapy: A Review"). Ce traitement est basé sur la production de radicaux libres par des molécules photosensibles, lors de l'exposition à de forts rayonnements UV ou LASER. En effet, les molécules activées transforment l'oxygène qui les entoure en radicaux libres qui sont des espèces hautement réactives produisant des dommages irréversibles dans les cellules. Les organes cellulaires principalement attaqués sont les mitochondries, les membranes cellulaire et nucléaire, les lysosomes etc..

Les molécules photosensibles sont injectées par voie intraveineuse et sont généralement retenues en concentration supérieure dans les tissus cancéreux. Ceci permet, après un certain temps, d'avoir une concentration dans les tissus à traiter plus importante que dans les tissus sains. Lorsque ces molécules sont exposées à la lumière (avec une longueur d'onde appropriée), elles produisent des radicaux libres à partir de l'oxygène, qui vont réagir avec des éléments vitaux de la cellule.

La thérapie photodynamique présente cependant certaines limites. En effet, les patients peuvent développer une certaine sensibilité à la lumière, ce qui limite le nombre d'applications de cette thérapie à un individu donné. D'autre part, les faibles longueurs d'ondes des rayonnements utilisés pour l'excitation des molécules photosensibles ne permettent pas de traverser une grande épaisseur de tissu, ce qui présente l'avantage d'être peu toxique pour les autres tissus, mais restreint l'indication aux cancers superficiels (peau et sub-cutanés). D'autres problèmes potentiels inhérents à l'utilisation de la thérapie photodynamique sont liés à la toxicité des molécules photosensibles et à la nécessité, dans certains cas, de l'utilisation d'oxygène pour « charger » les tissus à traiter.

Une autre approche utilisant des particules de $TiO_2$ a montré qu'il était possible de générer des radicaux libres à partir de molécules d'eau et d'oxygène sous une excitation UV [Shibata et al., Bioscience Biotechnology and Biochemistry 62:2306-2311 (1998)]. Cette approche a été utilisée sur des modèles in vitro et in vivo de cancer de la vessie. Une approche différente utilisant des microsphères radioactives de $Y_2O_3$ enrobées de silice, alumine ou oxide titane a été dé-crite dans le brevet EP-A-1162626 et sert à cibler les cellules cancéreuses.

Une autre approche, basée sur l'utilisation de particules activables par application d'un champ magnétique, a été décrite dans le brevet No. US6,514,481.La présente invention décrit une nouvelle classe de particules, désignées NanoXRay, utilisables en thérapie photo-dynamique. La présente invention décrit notamment de nouvelles particules, activables par des rayons X et/ou par des UV, capables d'induire une réponse thérapeutique ou diagnostique in vivo, de manière ciblée, même dans des tissus profonds.

La présente demande offre donc ainsi de nouveaux composés utilisables en thérapeutique et/ou en diagnostique (par exemple en imagerie), notamment chez l'homme. Les particules de l'invention peuvent être mises en oeuvre pour marquer, altérer ou détruire des cellules, tissus, ou organes, si nécessaire de manière ciblée, en combinaison avec une source de rayons X et/ou de rayons UV. Les particules de l'invention sont applicables à tout type de tissu, superficiel ou profond, chez tout organisme mammifère.

Un premier aspect de l'invention concerne donc des particules ou agrégats nanoparticulaires composites, capables de générer des radicaux libres et/ou de la chaleur sous excitation par des Rayons X et/ou UV.

Selon un autre aspect, l'invention concerne toute particule ou tout agrégat nanoparticulaire composé d'au moins deux composés inorganiques (de compositions distinctes), pouvant être traitée en surface afin de cibler spécifiquement des cellules ou des tissus biologiques, et ayant pour action de perturber ou de modifier un tissu biologique et/ou une cellule sous l'effet d'une source d'excitation.

Un objet plus particulier de l'invention réside dans une particule ou un agrégat nanoparticulaire composite biocompatible (capable de générer des radicaux libres ou de la chaleur sous excitation par des rayons X), comprenant :

- un noyau comprenant un premier composé inorganique absorbant les rayons X et émettant de l'énergie UV-visible, et un second composé, inorganique ou organique, absorbant de l'énergie UV-visible et produisant des radicaux libres au contact de l'eau ou de l'oxygène, et
- de manière facultative, un enrobage biocompatible.

Un autre objet de l'invention concerne un procédé de préparation de particules ou agrégats tels que définis ci-dessus.

Un autre objet de l'invention réside dans des compositions, pharmaceutiques ou diagnostiques, comprenant des particules ou agrégats tels que définis ci-dessus ou susceptibles d'être obtenus par le procédé ci-dessus.

Un autre objet de l'invention réside dans l'utilisation des compositions, particules ou agrégats tels que définis ci-dessus, pour le marquage ou la destruction de cellules, tissus ou organes, in vitro, ex vivo ou in vivo, ainsi que dans des méthodes correspondantes.

Au sens de l'invention, on entend par "particule" ou "agrégat nanoparticulaire" composite des produits complexes, synthétiques, de taille réduite. Leur forme peut être variée, par exemple arrondie, aplatie, allongée, sphérique, ovale, etc. La forme peut être déterminée ou contrôlée par le procédé de fabrication, et adaptée par l'homme du métier selon les applications recherchées.

La forme des particules n'a pas une grande influence sur leurs propriétés, notamment sur le rendement de la production de radicaux libres. Cependant, la forme peut influencer la « biocompatibilité » des particules. Ainsi, pour des raisons de pharmacocinétique, on préfère des particules ou agrégats nanoparticulaires de forme essentiellement sphérique ou arrondie. On préfère d'autre part des particules ou agrégats nanoparticulaires de forme assez homogène.

De manière préférée, la taille des particules ou agrégats nanoparticulaires selon l'invention est typiquement comprise entre 4 et 250 nm environ. Pour des applications in vivo chez l'homme ou l'animal, on préfère tout particulièrement des particules ou agrégats nanoparticulaires dont la taille est comprise entre 4 et 100 nm, encore plus préférentiellement entre 4 et 50 nm. En effet, la taille des objets doit idéalement être suffisamment petite pour leur permettre de diffuser dans l'organisme (tissus, cellules, vaisseaux sanguins, etc.), essentiellement sans être captés par les macrophages (phagocytose) et sans provoquer d'obstruction significative. De tels effets peuvent être obtenus avantageusement chez l'homme avec des particules d'une taille inférieure à 100 nm, préférentiellement inférieure à 50 nm.

Les particules ou agrégats selon l'invention doivent être biocompatibles, c'est-à-dire pouvoir être administrés à un organisme, typiquement un mammifère. Ce caractère biocompatible peut être assuré par exemple par la nature des composés constitutifs de la particule et/ou de l'enrobage éventuel.

Comme indiqué précédemment, les particules selon l'invention comprennent au moins deux types de composés inorganiques ayant des propriétés particulières, éventuellement recouverts d'un enrobage.

Le premier composé formant le noyau de la particule est un composé (ou un mélange de composés) inorganique (s) absorbant les rayons X et émettant de l'énergie UV-visible. La fonction principale de ce matériau est d'absorber les rayons X et de générer de l'énergie UV-visible, en particulier des UV. On utilise avantageusement un composé inorganique sous forme d'oxyde, hydroxyde, oxysulfure ou de sel, préférentiellement dopé par un agent dopant, de préférence choisi parmi les terres rares. Les propriétés de ce premier composé peuvent être ajustées par l'homme du métier en fonction du type de dopant utilisé, de la configuration électronique et de l'environnement cristallin autour de ce dopant, ainsi que de sa concentration. De manière particulièrement préférée, le dopant est choisi parmi les terres rares, de préférence à une concentration en cations (dopant) inférieure ou égale à environ 15% en solution solide. Ce % correspond au rapport de concentration des cations de terre rare sur les cations du composé inorganique.

Dans un mode de mise en oeuvre particulier, l'invention concerne une particule ou un agrégat nanoparticulaire tel que défini précédemment, dans lequel le premier composé inorganique est choisi parmi les oxydes et les hydroxydes dopés avec une terre rare, de préférence à une concentration inférieure à 15% en solution solide, ainsi que les composés mixtes d'oxydes de Ge, Hf et/ou Zr, dopés ou non, de préférence dopés à l'aide de cations de terre rare.

Le premier composé inorganique (ou composé principal) peut être choisi avantageusement parmi les composés suivants : $Y_2O_3$, $(Y,Gd)_2O_3$, $CaWO_4$, $GdO_2S$, $LaOBr$, $YTaO_3$, $BaFCl$, $Gd_2O_2S$, $Gd_3Ga_5O_{12}$, $Rb_3Lu(PO_4)_2$, $HfGeO_4$, et $Cs_3Lu(PO_4)_2$. Des composés particulièrement préférés dans le cadre de l'invention sont les oxydes $Y_2O_3$ et $HfGeO_4$.

Le dopant utilisé est avantageusement une terre rare choisie par exemple parmi Gd, Eu, Tb, Er, Nb, Pr et Ce. Des dopants particulièrement préférés sont Gd, Eu et Tb.

Dans un exemple spécifique de particule selon l'invention, le premier composé inorganique est choisi parmi $Y_2O_3$ dopé avec Gd, Eu ou Tb.

Dans un second exemple spécifique de particule selon l'invention, le premier composé inorganique est le $HfGeO_4$ dopé ou non, de préférence dopé, ou le $HfGeO_4$ en solution mixte avec Zr (qui peut représenter jusqu'à 50% de la solution mixte).

Il est entendu que d'autres composés inorganiques, oxydes, hydroxydes, oxysulfures ou sels et dopants peuvent être envisagés par l'homme du métier pour la réalisation de particules selon l'invention. En outre, plusieurs oxydes, hydroxydes, oxysulfures ou sels et/ou dopants peuvent être utilisés en mélange dans une même particule de l'invention.

Le second composé formant le noyau de la particule est un composé (ou un mélange de composés) inorganique ou organique absorbant de l'énergie UV-visible et produisant des radicaux libres au contact de l'eau ou de l'oxygène. La fonction principale de ce matériau est d'absorber l'énergie UV-visible, notamment les UV, et de transformer l'eau (ou $O_2$) présente à la surface de ce composé en radicaux libres par une réaction de type photo-catalytique.

Le second composé est avantageusement un composé inorganique, qui peut être choisi parmi les composés semi-conducteurs, tels que notamment le $TiO_2$, le ZnO et, de manière non restrictive, CdS, CdSe, CdTe, MnTe et des solutions mixtes (par exemple CdZnSe, CdMnSe, etc.), éventuellement dopés (comme décrit pour le premier composé inorganique).

Dans un mode particulier de mise en oeuvre, on utilise comme second composé inorganique $TiO_2$, avantageusement sous forme anatase, et éventuellement dopé.

Dans une autre variante de réalisation, le second composé peut être une molécule organique absorbant dans la région de l'UV et générant des radicaux libres en présence d'oxygène (par exemple certaines molécules utilisées en thérapie photodynamique). On préfère toutefois utiliser comme second composé un composé inorganique.

Dans le noyau des particules de l'invention, les composés inorganiques (et éventuellement organiques) peuvent être agencés ou organisés de différentes façons.

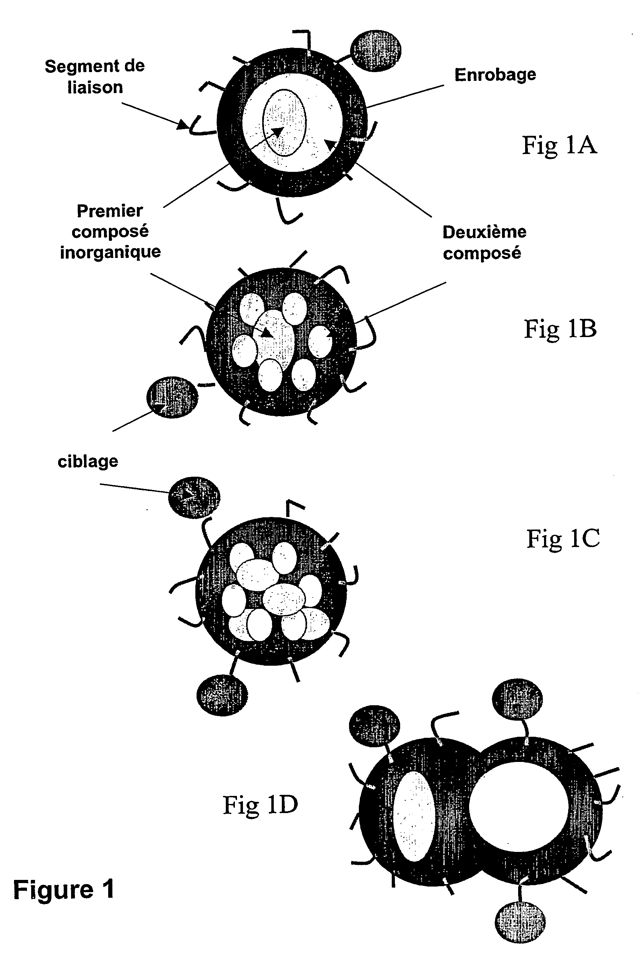

Ainsi, dans une première variante de mise en oeuvre, le premier composé inorganique forme le coeur du noyau, et le second composé (inorganique ou organique) se présente sous forme d'une couche ou de nanoparticules à la surface du coeur (voir Figure 1A et 1B).

Dans une variante de mise en oeuvre spécifique, les deux composés inorganiques du noyau sont disposés en multicouches successives, le premier composé inorganique formant préférentiellement la couche interne (le coeur).

Ainsi, une forme de réalisation préférée de l'invention consiste en des particules dont le noyau comprend un coeur constitué par le premier composé inorganique, recouvert d'une couche formée par le second composé (Figure 1A). Le coeur du noyau formé par le premier composé inorganique présente typiquement une dimension comprise entre 5 et 50 nm environ, par exemple entre 7 et 40 nm, et/ou la couche formée par le second composé à la surface du coeur possède une épaisseur comprise typiquement entre 1 et 30 nm environ, par exemple entre 2 et 25 nm.

Dans une autre variante de mise en oeuvre, les deux composés du noyau sont présents sous forme d'un mélange de nanoparticules (Figure 1C). De telles nanoparticules peuvent être de taille et de forme variées. D'une manière générale, à titre indicatif, les nanoparticules présentent une taille comprise entre 3 et 100 nm environ et préférentiellement entre 5 à 25 nm.

Dans une autre variante de mise en oeuvre, les deux composés inorganiques du noyau sont présents sous forme de deux noyaux au contact l'un de l'autre (Figure 1D).

De manière générale, l'efficacité ou les propriétés des particules peuvent être adaptées par l'homme du métier en jouant sur la quantité relative des deux types de composés, le recouvrement entre les spectres d'émission et d'absorption des deux composés inorganiques, la structure cristalline des matériaux, la surface de contact entre le second composé et l'eau et/ou la distance entre le premier et le second composés.

S'agissant de la quantité relative des deux composés, on préfère typiquement des particules dans lesquelles les deux composés sont présents en des quantités similaires. La rapport de la quantité ou de la concentration du premier composé sur le second composé peut toutefois être ajusté par l'homme du métier, de préférence dans une gamme allant de 0,1 à 10, plus préférentiellement de 0,2 à 5.

Par ailleurs, les expériences réalisées par les inventeurs montrent que plus le recouvrement entre le spectre d'émission du premier composé inorganique (matériau de coeur) et le spectre d'absorption du second composé est important, plus le rendement des particules est important.

De même, plus la surface de contact entre le second composé (par exemple $TiO_2$) et l'eau est importante, plus le rendement est important.

D'autre part, le rendement du transfert d'énergie dans les particules dépend également de la distance entre le premier composé inorganique (matériau de coeur) et le second. Plus cette distance est courte et/ou plus la surface de contact est importante, plus le transfert d'énergie est efficace et plus la particule est active.

L'homme du métier peut donc adapter les propriétés des particules en faisant varier les paramètres mentionnés ci-dessus, par exemple en fonction des utilisations envisagées (diagnostic, thérapeutique, etc.).

Il est entendu que les particules de l'invention peuvent comprendre, outre les deux types de composés décrits précédemment, d'autres molécules, composés ou matériaux de structure ou de surface, destinés à améliorer leur stabilité, propriété, fonction, spécificité, etc.

Ainsi, comme indiqué précédemment, les particules ou agrégats nanoparticulaires selon l'invention peuvent comprendre en outre un élément de surface permettant de cibler spécifiquement des cellules ou des tissus biologiques. Cet élément de surface peut être lié aux particules par tout moyen, de préférence covalent, éventuellement par l'intermédiaire d'un segment de liaison. Il peut être associé à l'un des composés inorganiques ou à l'enrobage éventuellement présent, comme il sera décrit dans la suite du texte.

L'élément de ciblage de surface peut être toute structure biologique ou chimique présentant une affinité pour des molécules présentes dans le corps humain ou animal. Il peut ainsi s'agir d'un peptide, polypeptide, nucléotide, polynucléotide, une hormone, une vitamine, etc. et, de manière générale, de tout ligand de molécules (par exemple récepteurs, marqueurs, antigènes, etc.). On peut mentionner à titre d'illustration des ligands de molécules exprimées par des cellules pathologiques, notamment des ligands d'antigènes tumoraux, de récepteurs hormonaux, de récepteurs de cytokines ou de récepteurs de facteurs de croissance, par exemple.

L'élément de ciblage permet, lorsqu'il est présent, de diriger préférentiellement les particules de l'invention vers des cellules, tissus ou organes d'intérêt, et ainsi de confiner l'action à ces tissus. Un tel ciblage est particulièrement utile lorsque les particules sont administrées par voie systémique, par exemple pour les tissus profonds.

Comme indiqué précédemment, les particules ou agrégats nanoparticulaires selon l'invention peuvent comprendre en outre un enrobage. Un tel enrobage permet avantageusement de préserver l'intégrité des particules in vivo, d'assurer ou d'améliorer leur biocompatibilité, et de faciliter leur fonctionnalisation (par exemple avec des molécules de liaison (« spacer »), des polymères biocompatibles, des agents de ciblage, des protéines, etc.).

L'enrobage peut être composé de toute structure amorphe ou cristalline. Pour préserver l'activité des particules de l'invention, il est souhaitable que l'enrobage permette la diffusion de petites molécules et de radicaux libres. En particulier, il est important que l'enrobage permette le passage de l'eau (ou $O_2$) et de sa forme radicalaire après transformation. Ceci peut être assuré en utilisant des matériaux présentant une certaine porosité et/ou une couche d'enrobage de faible épaisseur et poreuse. Ainsi par exemple, on utilise typiquement un enrobage possédant une porosité comprise entre 0,2 et 10 nm. L'enrobage possède par ailleurs une épaisseur comprise généralement entre 0,1 et 50 nm environ, par exemple entre 0,2 et 40 nm.

De façon générale, l'enrobage peut être non-biodégradable ou biodégradable. Pour les enrobages non-biodégradables, on utilise par exemple un ou plusieurs matériaux choisis parmi la silice, l'agarose, l'alumine, un polymère carboné saturé ou un polymère inorganique, réticulé ou non, modifié

ou non (le polystyrène par exemple). Pour les enrobages biodégradables, on utilise par exemple un ou plusieurs matériaux choisis parmi des molécules biologiques modifiées ou non, naturelles ou non, un polymère de molécule biologique modifiée ou non, de forme naturelle ou non, ou un polymère biologique, tel que le saccharide, un oligosaccharide, un polysaccharide, polysulfaté ou non, par exemple le dextran. Les matériaux ou composés ainsi mentionnés peuvent être utilisés seuls ou en mélanges ou en assemblages, composites ou non, covalents ou non, éventuellement en combinaison avec d'autres composés. D'autre part, on peut également utiliser tout matériau mentionné ci-dessus, hydro- ou liposoluble, de façon naturelle ou artificielle.

L'enrobage comprend de préférence un ou des composés choisi parmi la silice ($SiO_2$), l'alumine, le Polyéthylène Glycol (PEG) ou le Dextran, éventuellement en mélange(s).

L'enrobage peut par ailleurs comporter différents groupes fonctionnels (ou segments de liaison), permettant la liaison à la surface de la particule de toute molécule d'intérêt.

Des groupes fonctionnels utiles sont par exemple $(CH_2)_n COOH$, dans lequel n est un entier allant de 1 à 10.

Les molécules couplées à la surface de la particule peuvent être, par exemple :

- l'agent de ciblage ;
- une molécule assurant ou améliorant la biocompatibilité ; ou
- une molécule permettant à la particule d'échapper au système immunitaire (et notamment d'éviter les interactions avec les macrophages et SRE).

Dans un mode de réalisation préféré, les particules ou agrégats nanoparticulaires selon l'invention comprennent un enrobage auquel l'élément de ciblage de surface est lié, de préférence par l'intermédiaire d'un segment de liaison.

Des particules ou agrégats préférés de l'invention comprennent, à titre de premier composé inorganique, $Y_2O_3$ dopé par une terre rare ou $HfGeO_4$ éventuellement dopé et/ou en solution mixte avec Zr, à titre de second composé inorganique, $TiO_2$ et, de préférence, un enrobage à base de $SiO_2$ ou de Dextran.

Des particules ou agrégats particuliers au sens de l'invention comprennent, à titre de premier composé inorganique, $Y_2O_3$:Gd, à titre de second composé inorganique, $TiO_2$ de structure anatase et, de préférence, un enrobage à base de $SiO_2$.

D'autres particules ou agrégats particuliers au sens de l'invention comprennent, à titre de premier composé inorganique, $Y_2O_3$:Tb, à titre de second composé inorganique, $TiO_2$ et, de préférence, un enrobage à base de Dextran.

Des exemples de particules sont notamment:

- Une particule ou un agrégat comprenant un coeur comprenant du $Y_2O_3$:Gd recouvert d'une couche de $TiO_2$, et un enrobage à base de $SiO_2$, de préférence fonctionnalisé. De préférence, le coeur possède une forme arrondie ou sphérique, de dimension comprise entre 5 et 50 nm environ (typiquement de l'ordre de 30nm), la couche de $TiO_2$ possède une épaisseur comprise entre 5 et 30 nm environ (typiquement de l'ordre de 5 nm), et l'enrobage possède une épaisseur comprise entre 1 et 50 nm environ (typiquement de l'ordre de 5 nm).
- Une particule ou un agrégat nanoparticulaire comprenant un noyau comprenant des microparticules de $Y_2O_3$:Tb et de $TiO_2$, et un enrobage à base de Dextran, de préférence fonctionnalisé.
- Une particule ou un agrégat comprenant un coeur comprenant du $HfGeO_4$ recouvert d'une couche de $TiO_2$, et un enrobage à base de $SiO_2$, de préférence fonctionnalisé.

Un autre objet de l'invention concerne un procédé de production de particules ou agrégats nanoparticulaires tels que définis précédemment, comprenant :

- le mélange des deux composés tels que définis précédemment pour former une particule ou un agrégat et, éventuellement
- l'enrobage de la particule ou agrégat.

Dans une variante de mise en oeuvre, le procédé comprend en outre une étape de fonctionnalisation, comprenant l'introduction sur la particule ou agrégat d'un élément de ciblage.

Les matériaux qui composent les particules ou agrégats de l'invention peuvent être produits par différentes techniques, qui sont connues en soi de l'homme du métier. Le procédé peut être adapté par l'homme du métier selon la nature des composés utilisés, et selon leur agencement dans les particules et agrégats. Ainsi, dans un mode de réalisation particulier, le procédé comprend:

- la préparation du coeur de la particule comprenant le premier composé inorganique,
- le recouvrement du coeur ainsi formé par une couche comprenant le second composé et, de manière préférée,
- l'enrobage de la particule ou agrégat ainsi obtenu par un matériau poreux.

Des méthodes alternatives de production de matériaux utilisables pour la production des particules de l'invention sont décrites par exemple dans Nelson et al, Chem. Mater. 2003, 15, 688-693 "Nanocrystalline Y2O3:Eu Phosphors Prepared by Alkalide Reduction" ou encore dans Liu et al., Journal of Magnetism and Magnetic Materials 270 (2004) 1-6 "Preparation and characterization of amino-silane modified superparamagnetic silica nanospheres".

Dans un exemple spécifique de réalisation, décrit en détail dans la partie expérimentale, permettant la production ou la synthèse d'une particule ou d'un agrégat comprenant un coeur comprenant du $Y_2O_3$:Eu recouvert d'une couche de $TiO_2$ et d'un enrobage à base de $SiO_2$, le procédé comprend les étapes suivantes :

- les nanoparticules du $Y_2O_3$:Eu sont fabriquées en utilisant une réduction avec $YCl_3$, $EuCl_3$ et éther couronne en milieu homogène,
- le revêtement avec $TiO_2$ peut être réalisé en précipitant $TiCl_4$ en solution acide, puis,
- la couche de silice facultative est obtenue par précipitation en milieu basique de silicate de sodium.

Dans un deuxième exemple spécifique de réalisation, décrit en détails dans la partie expérimentale, permettant la production ou la synthèse d'une particule ou d'un agrégat comprenant un coeur comprenant du $HfGeO_4$ recouvert d'une couche de $TiO_2$ et d'un enrobage à base de $SiO_2$, le procédé comprend les étapes suivantes :

- la synthèse du coeur par co-précipitation des sels d'hafnium et de germanium amorphe,
- l'enrobage par $TiO_2$, et
- l'enrobage par $SIO_2$ a partir de TEOS et/ou de silicate de sodium.

Un autre objet de l'invention réside dans toute composition comprenant des particules ou agrégats tels que définis précédemment et/ou susceptibles d'être obtenus par le procédé décrit précédemment. Bien que cela ne soit pas obligatoire, dans les compositions de l'invention, les particules présentent avantageusement une forme et une taille assez homogènes. Généralement, les compositions comprennent entre 0,3 et 3000 mg de particules pour 100 ml. Les compositions peuvent être sous forme solide, liquide (particules en suspension), de gel, pâte, etc.

Un objet particulier de l'invention concerne une composition pharmaceutique comprenant des particules ou agrégats nanoparticulaires tels que définis précédemment et, éventuellement, un excipient ou véhicule acceptable sur le plan pharmaceutique.

Un autre objet particulier de l'invention concerne une composition diagnostique ou d'imagerie comprenant des particules ou agrégats nanoparticulaires tels que définis précédemment et, éventuellement, un excipient ou véhicule acceptable sur le plan physiologique.

L'excipient ou véhicule mis en oeuvre peut être tout support habituel pour ce type d'applications, comme par exemple des solutions salines, isotoniques, stériles, tamponnées, etc. Elles peuvent comprendre en outre des agents de stabilisation, des agents édulcorants, tensio-actifs, etc. Elles peuvent être formulées sous forme d'ampoules, de flacons, de comprimés, de gélules, en utilisant des techniques de galénique connues en soi.

Les compositions, particules et agrégats de l'invention peuvent être utilisés dans de nombreux domaines, particulièrement en médecine humaine ou animale. Sous l'effet de rayons X, le coeur de la nanoparticule est excité et produit de l'énergie UV-visible, en particulier des UV. Les UV excitent le second composé qui, au contact de l'eau, émet des radicaux libres. Selon le temps d'exposition à la source d'excitation, les particules peuvent ainsi permettre la destruction de tissus (temps d'exposition par exemple de plusieurs minutes, par exemple 5 minutes) ou, simplement, une visualisation (imagerie, diagnostic: temps d'exposition très courts : de l'ordre de quelques secondes). Grâce à la forte pénétration des rayons X, les particules de l'invention sont applicables pour un balayage de tout tissu dans le corps. Elles peuvent également être utilisées avec une source d'excitation UV, pour les tissus de surface ou des cavités (peau, vessie, poumon, colon, etc.).

Un objet particulier de l'invention réside ainsi dans la destruction de cellules cibles.

Un autre objet de l'invention réside dans l'utilisation de compositions, particules ou agrégats nanoparticulaires tels que définis précédemment, en combinaison avec les rayons UV, pour la fabrication d'un médicament destiné à la destruction de cellules cibles superficielles ou des cavités.

Un autre objet particulier de l'invention réside dans l'utilisation de compositions, particules ou agrégats nanoparticulaires tels que définis précédemment, en combinaison avec les rayons X, pour la fabrication d'un médicament destiné à induire ou causer la lyse ou la destruction de cellules cibles, in vitro, ex vivo ou in vivo, comprenant la mise en contact de cellules cibles avec une ou des particules ou agrégats nanoparticulaires tels que définis précédemment, pendant une période de temps suffisante pour permettre aux particules ou agrégats de pénétrer dans des cellules cibles et, l'exposition des cellules à des rayons X ou UV, ladite exposition induisant ou causant la lyse ou la destruction desdites cellules cibles.

Les cellules cibles peuvent être toutes cellules pathologiques, c'est-à-dire des cellules impliquées dans un mécanisme pathologique, par exemple des cellules prolifératives, telles que des cellules tumorales, sténosantes (cellules du muscle lisse), ou du système immunitaire (clones de cellules pathologiques). Une application préférée réside dans le traitement (par exemple la destruction ou l'altération des fonctions) de cellules cancéreuses.

A cet égard, un objet particulier de l'invention réside dans l'utilisation de compositions, particules ou agrégats nanoparticulaires tels que définis précédemment (en combinaison avec des rayons X ou UV) pour la fabrication d'un médicament destiné au traitement du cancer.

Un autre objet particulier de l'invention réside dans l'utilisation de compositions, particules ou agrégats nanoparticulaires tels que définis précédemment, en combinaison avec les rayons X, pour la fabrication d'un médicament destiné à induire ou causer la lyse ou la destruction de cellules cancéreuses, in vitro, ex vivo ou in vivo, comprenant la mise en contact de cellules cancéreuses avec une ou des particules ou agrégats nanoparticulaires tels que définis précédemment, pendant une période de temps suffisante pour permettre aux particules ou agrégats de pénétrer dans les cellules cancéreuses et, l'exposition des cellules à des rayons X ou UV, ladite exposition induisant ou causant la lyse ou la destruction desdites cellules.

Un autre objet de l'invention concerne le traitement du cancer, comprenant l'administration à un patient atteint d'un cancer d'une composition ou de particules ou agrégats nanoparticulaires tels que définis précédemment, dans des conditions permettant aux particules ou agrégats nanoparticulaires de pénétrer dans les cellules cancéreuses, et le traitement ultérieur du patient en présence d'une source d'excitation choisie parmi les rayons X et les UV, conduisant à une altération, une perturbation ou une destruction fonctionnelle de cellules cancéreuses du patient, traitant ainsi le cancer.

L'invention est utilisable pour traiter tout type de cancer, notamment les tumeurs solides, métastasées ou non, par exemple choisies parmi les cancers du poumon, foie, rein, vessie, sein, tête-et-cou, cerveau, ovaires, prostate, peau, intestin, colon, etc.

Les rayons peuvent être appliqués à tout moment après l'administration des particules, en une ou plusieurs fois, en utilisant tout système de radiothérapie ou de radiographie déjà disponible. Les particules peuvent être administrées par différentes voies, de préférence par injection, systémique ou locale, ou de manière orale. Des injections ou administrations répétées peuvent être envisagées, si nécessaire.

De façon générale et non restrictive, les rayonnements suivants peuvent être appliqués dans différents cas pour exciter les particules :

- Rayons X superficiels (20 à 50 keV) : pour l'excitation de nanoparticules en superficie (pénétration de quelques millimètres).
- Rayons X pour le diagnostic 50 à 150 keV.
- Rayons X (ortho voltage) de 200 à 500 keV permettant de pénétrer des épaisseurs de tissus jusqu'à 6 cm.
- Rayons X (méga voltage) de 1000 keV à 25000 keV. Pour exemple l'excitation de nanoparticules pour le traitement du cancer de la prostate peut se faire via 5 rayons X focalisés ayant une énergie de 15000 keV.

Dans le domaine diagnostic, les particules de l'invention sont utilisables comme agent de contraste, pour détecter et/ou visualiser tout type de tissu. Ainsi, un objet de l'invention concerne l'utilisation de compositions, particules ou agrégats nanoparticulaires tels que définis précédemment, en combinaison avec les rayons X, pour la fabrication d'une composition destinée à la détection ou à la visualisation de cellules, tissus ou organes.

Un autre objet de l'invention réside dans l'utilisation de compositions, particules ou agrégats nanoparticulaires tels que définis précédemment, en combinaison avec les rayons UV, pour la fabrication d'une composition destinée à la détection ou à la visualisation de cellules, tissus ou organes superficiels ou des cavités.

Le terme "en combinaison" indique que l'effet recherché est obtenu lorsque les cellules, tissus ou organes d'intérêt, ayant incorporé en partie des nanoparticules de l'invention, sont excités par la source déterminée. Toutefois, il n'est pas nécessaire que les particules et les rayons soient administrés simultanément, ni selon le même protocole.

Le terme "traitement" désigne toute amélioration des signes pathologiques, comme notamment une diminution de la taille ou du développement d'une tumeur ou d'une zone tissulaire pathologique, la suppression ou la destruction de cellules ou tissus pathologiques, un ralentissement de la progression de la pathologie, une réduction de la formation de métastases, une régression ou une rémission complète, etc.

Les particules de l'invention peuvent également être utilisées in vitro ou ex vivo.

D'autres aspects et avantages de l'invention apparaîtront à la lecture des exemples qui suivent, qui doivent être considérés comme illustratifs et non limitatifs.

Légende des Figures

La Figure 1 donne une représentation schématique de la structure de particules de l'invention.

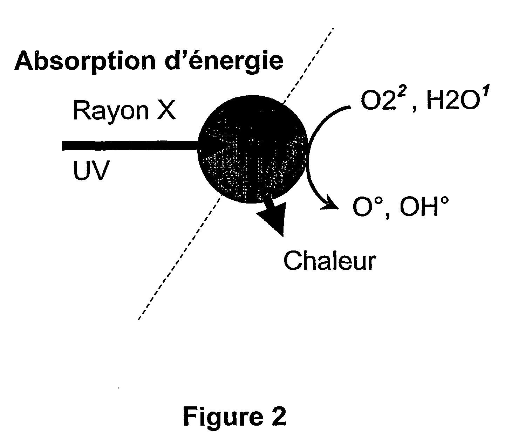

La Figure 2 représente le mode d'activation de particules selon l'invention en présence d'une source de rayons X.

La Figure 3 : Cliché de microscopie électronique à transmission montrant des nanoparticules de $Y_2O_3$ dopées au Gadolinium.

La Figure 4 présente le taux de survie des cellules après incubation avec les nanoparticules

La Figure 5 présente une image de microscopie confocale laser montrant l'accumulation des nanoparticules (en gris sur le cliché) dans les membranes de la cellule.

Exemples

### 1. Production de nanoparticules de $Y_2O_3$ dopées avec de l'Erbium ou du Gadolinium

Des nanoparticules de $Y_2O_3$ dopées avec de l'Erbium ou du Gadolinium ont été synthétisées à partir de surfactants fonctionnalisés (Y-AOT3, Eu-AOT3, et Gd-AOT3). Un mélange approprié des surfactants (en fonction de la concentration finale désirée [Eu-AOT3]/[Y-AOT3]=0,01 ; 0,05 ; 0,1 ; 0,15) a été dispersé dans de l'isooctane ou du cyclohexane, et de l'eau a été ajoutée afin de permettre la formation de micelles. La taille des micelles, qui influence la taille des matériaux obtenus, est contrôlée par la quantité d'eau ajoutée au mélange. La formation de l'hydroxyde se fait par addition de base. Les particules ont ensuite été lavées (avec un mélange eau/éthanol), séchées puis chauffées à 800°C afin de former des nanoparticules cristallines.

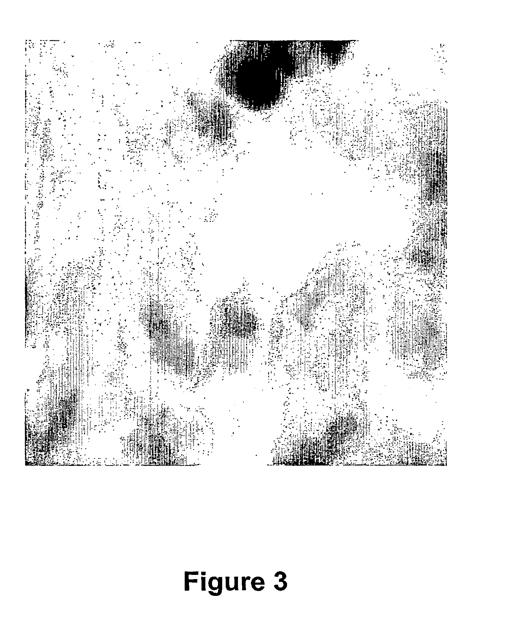

10 mg de nanoparticules ont été dispersés dans 50 ml d'eau à pH=7,5. Une goutte de cette dispersion a été déposée sur une grille de cuivre/carbone et observée par microscopie électronique à transmission. Une photographie prise par microscopie est présentée sur la figure 3.

Les composés fabriqués ont montré une fluorescence UV sous excitation à l'aide de rayons X (pour $Y_2O_3$ dopé avec Gd).

### 2. Production de nanoparticules de $HfGeO_4$ enrobées d'oxyde de titane et de silice

La synthèse du matériau de coeur, $HfGeO_4$, se fait par simple co-précipitation de sels d'hafnium ($HfOCl_2$ , 8 $H_2O$) et de germanium amorphe ($GeO_2$) en milieu aqueux. Un traitement thermique d'une durée comprise entre 4h et 10h à 1100°C ou à une température inférieure assure ensuite la cristallisation de $HfGeO_4$ sous forme de nanoparticules.

L'enrobage de ces particules à l'aide de $TiO_2$ se fait via l'utilisation du précurseur de titane $TiCl_4$. Sa réaction avec la soude conduit en effet à la condensation de $TiO(OH)_2$ à la surface de $HfGeO_4$. Un traitement thermique à 500°C d'une durée comprise entre 1 h30 et 3h00 assure ensuite le passage de $TiO_2$ sous une forme cristalline, anatase, dotée de propriétés photocatalytiques. L'enrobage à l'aide de silice s'effectue à partir de TEOS. L'hydrolyse lente et contrôlée de TEOS en milieu alcoolique et ammoniaqué conduit à la formation d'une couche de silice à la surface des particules.

### 3. Biocompatibilité des nanoparticules

La biocompatibilité et la non-toxicité in vitro des nanoparticules enrobées par de la silice ont été testées in vitro sur des lignées cellulaires MCF7, KB et UCI. Les nanoparticules (30 à 1000 pg de particules / 1000 cellules) ont été incubées avec ces cellules pendant 24, 48 et 72 h. Le taux de survie des cellules a été mesuré comme suit :

$$Rsur = \frac{\text{nombre de cellules vivante (avec particules) /nombre de cellule mortes (avec particules)}}{\text{nombre de cellules vivante (sans particules) /nombre de cellule mortes (sans particules)}}$$

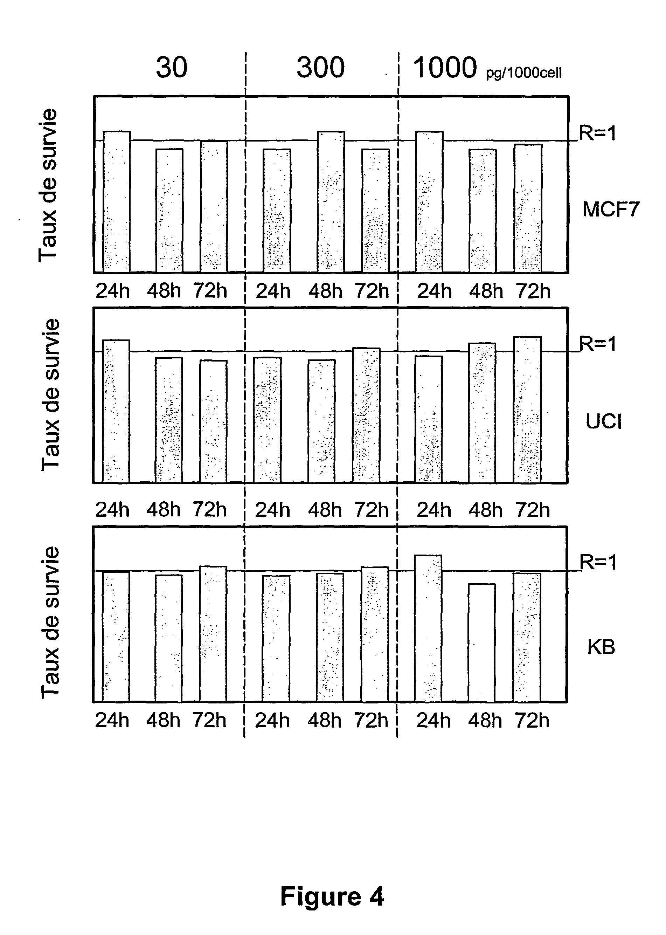

Aucune différence significative n'a été observée avec les témoins sur le taux de survie et la division cellulaire (Figure 4).

**4. Ciblage et internalisation des nanoparticules**

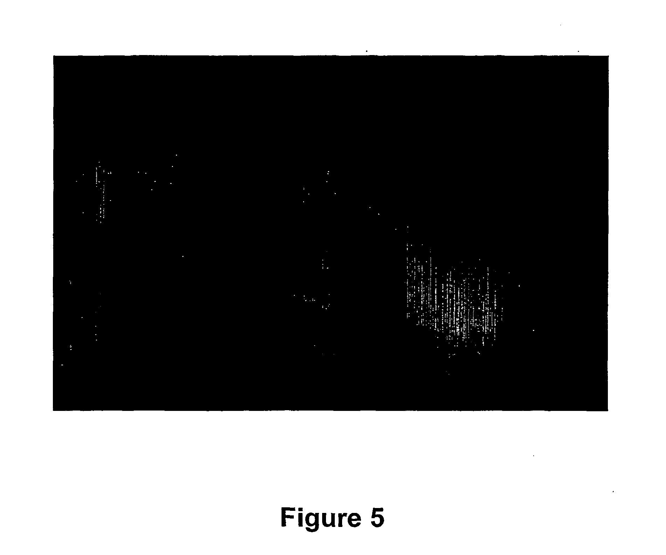

Le ciblage et l'entrée spécifique des particules (avec éléments de ciblage en surface) dans les cellules via des récepteurs de surface ont été observés par microscopie confocale laser. Des nanoparticules ont été recouvertes de silice et fonctionnalisées avec LHRH via une liaison chimique (comme décrit dans Lévy et al, Chem. Mater.; 2002; 14 (9) pp 3715; Nanochemistry: "*Synthesis and Characterization of Multifunctional Nanoclinics for Biological Applications*"). Les nanoparticules ont été incubées pendant 24 heures avec des cellules MCF7 (portant le récepteur LHRH) et observées par microscopie confocale laser. La figure 5 présente un image enregistrée après 24h et montre une accumulation des nanoparticules dans les membranes et dans les noyaux des cellules.

**5. Protocole d'administration chez l'animal et traitement.**

Les nanoparticules sont dispersées dans une solution isotonique (PBS, Saline) à une concentration allant de 1 à 20 mg/ml. Des injections de 0,5ml sont faites par voie intraveineuse, intratumorale ou intrapéritonéale. 24 à 48 heures post injection, les animaux sont soumis à des rayons X :

- Corps entier pour le diagnostic ou le traitement des métastases. Les rayons X utilisés peuvent être générés par les équipements de radiographie usuels ;
- Focalisés pour le traitement d'une tumeur solide ou d'une zone particulière du corps.

Des stratégies complémentaires peuvent être appliquées :

- après une injection simple, de multiples expositions aux Rayons X sont réalisées ;
- de multiples injections (espacées de plusieurs semaines), à chaque fois suivies d'une exposition simple ou multiple ;
- de multiples injections (espacées de plusieurs jours), suivie d'une exposition simple ou multiple.

**Revendications**

**1.** Particule ou agrégat nanoparticulaire composite biocompatible, capable de générer des radicaux libres ou de la chaleur sous excitation par des rayons X, comprenant :

- un noyau comprenant un premier composé inorganique absorbant les rayons X et émettant de l'énergie UV-visible, et un second composé, inorganique ou organique, absorbant de l'énergie UV-visible et produisant des radicaux libres au contact de l'eau ou de l'oxygène, et
- de manière facultative, un enrobage biocompatible.

**2.** Particule ou agrégat nanoparticulaire selon la revendication 1, **caractérisé en ce que** sa taille est comprise entre 4 et 250 nm, de préférence entre 4 et 100 nm, encore plus préférentiellement entre 4 et 50 nm.

**3.** Particule ou agrégat nanoparticulaire selon l'une des revendications 1 à 2, **caractérisé en ce que** le premier composé inorganique est sous forme d'oxyde, hydroxyde, oxysulfure ou de sel, dopé avec une terre rare, de préférence à une concentration en cations inférieure à 15% en solution solide.

**4.** Particule ou agrégat nanoparticulaire selon l'une des revendications 1 à 3, **caractérisé en ce que** le premier composé inorganique est choisi parmi $Y_2O_3$, $(Y,Gd)_2O_3$, $CaWO_4$, $GdO_2S$, $LaOBr$, $YTaO_3$, $BaFCl$, $Gd_2O_2S$, $Gd_3Ga_5O_{12}$, $HfGeO_4$, $Rb_3Lu(PO_4)_2$ et $Cs_3Lu(PO_4)_2$, dopé avec une terre rare choisie parmi Gd, Eu, Tb, Er, Nb, Pr et Ce.

**5.** Particule ou agrégat nanoparticulaire selon la revendication 4, **caractérisé en ce que** le premier composé inorganique est choisi parmi $Y_2O_3$ dopé avec Gd, Eu ou Tb.

**6.** Particule ou agrégat nanoparticulaire selon la revendication 4, **caractérisé en ce que** le premier composé inorganique est le $HfGeO_4$ en solution mixte avec Zr.

**7.** Particule ou agrégat nanoparticulaire selon l'une des revendications 1 à 6, **caractérisé en ce que** le second composé est un composé inorganique choisi parmi les composés semi-conducteurs, de préférence $TiO2$, ZnO, CdS, CdSe, CdTe, MnTe et des solutions mixtes, éventuellement dopés.

**8.** Particule ou agrégat nanoparticulaire selon l'une quelconque des revendications précédentes, **caractérisé en en ce que** le premier composé inorganique forme le coeur du noyau, et en ce que le second composé se présente sous forme d'une couche ou de nanoparticules à la surface du coeur.

**9.** Particule ou agrégat nanoparticulaire selon l'une quelconque des revendications précédentes, **caractérisé en en ce que** les deux composés inorganiques du noyau sont disposés en multicouches successives.

**10.** Particule ou agrégat nanoparticulaire selon la revendication 9, **caractérisé en ce que** le coeur du noyau formé par le premier composé inorganique présente une dimension comprise entre 5 et 50 nm environ, et **en ce que** la couche formée par le second composé à la surface du coeur possède une épaisseur comprise entre 1 et 30 nm environ.

**11.** Particule ou agrégat nanoparticulaire selon l'une quelconque des revendications 1 à 8, **caractérisé en en ce que** les deux composés du noyau sont présents sous forme d'un mélange de nanoparticules.

**12.** Particule ou agrégat nanoparticulaire selon l'une quelconque des revendications précédentes, **caractérisé en en ce que** le rapport de la quantité ou de la concentration du premier composé sur le second composé est compris entre 0,2 et 5.

**13.** Particule ou agrégat nanoparticulaire selon l'une quelconque des revendications précédentes, **caractérisé en en ce qu'**il comprend en outre un élément de surface permettant de cibler spécifiquement des cellules ou des tissus biologiques.

**14.** Particule ou agrégat nanoparticulaire selon l'une quelconque des revendications précédentes, **caractérisé en ce qu'**il comporte un enrobage permettant la diffusion de petites molécules et de radicaux libres.

**15.** Particule ou agrégat nanoparticulaire selon la revendication 14, **caractérisé en ce que** l'enrobage est composé d'une structure amorphe ou cristalline poreuse, de préférence il comprend un composé choisi parmi la silice, l'alumine, le PEG et le Dextran.

**16.** Particule ou agrégat nanoparticulaire selon l'une des revendications 13 à 15, **caractérisé en en ce que** l'élément de surface permettant de cibler spécifiquement des cellules ou des tissus biologiques est lié à l'enrobage.

**17.** Particule ou agrégat nanoparticulaire selon l'une des revendications 13 à 16, **caractérisé en ce que** l'élément de ciblage de surface est une structure biologique ou chimique présentant une affinité pour des molécules présentes dans le corps humain ou animal, tel qu'un peptide, polypeptide, nucléotide, polynucléotide, une hormone ou une vitamine.

**18.** Particule ou agrégat nanoparticulaire, **caractérisé en ce qu'**il comprend un coeur comprenant du $Y_2O_3$:Gd recouvert d'une couche de $TiO_2$, et un enrobage à base de $SiO_2$.

**19.** Particule ou agrégat nanoparticulaire, **caractérisé en ce qu'**il comprend un noyau comprenant des microparticules de $Y_2O_3$:Tb et de $TiO_2$, et un enrobage à base de Dextran.

**20.** Particule ou agrégat nanoparticulaire selon l'une quelconque des revendications précédentes, **caractérisé en ce que** sa forme est essentiellement sphérique.

**21.** Procédé de production de particules ou agrégats nanoparticulaires selon l'une quelconque des revendications 1 à 20, comprenant :

- le mélange des deux composés tels que définis dans les revendications 1 à 20 pour former une particule ou un agrégat et, éventuellement
- l'enrobage de la particule ou agrégat.

**22.** Procédé selon la revendication 21, **caractérisé en ce qu'**il comprend:

- la préparation du coeur de la particule comprenant le premier composé inorganique,
- le recouvrement du coeur ainsi formé par une couche comprenant le second composé et, de manière préférée,
- l'enrobage de la particule ou agrégat ainsi obtenu par un matériau poreux.

**23.** Composition pharmaceutique ou diagnostique comprenant des particules ou agrégats nanoparticulaires selon l'une quelconque des revendications 1 à 20.

**24.** Utilisation de particules ou agrégats nanoparticulaires selon l'une quelconque des revendications 1 à 20, ou d'une composition selon la revendication 23, en combinaison avec les rayons X, pour la fabrication d'un médicament destiné à la destruction de cellules cibles.

**25.** Utilisation de particules ou agrégats nanoparticulaires selon l'une quelconque des revendications 1 à 20, ou d'une composition selon la revendication 23, en combinaison avec les rayons UV, pour la fabrication d'un médicament destiné à la destruction de cellules cibles superficielles ou des cavités.

**26.** Utilisation de particules ou agrégats nanoparticulaires selon l'une quelconque des revendications 1 à 20, ou d'une composition selon la revendication 23, en combinaison avec les rayons X ou UV, pour la fabrication d'un agent destiné à la détection ou à la visualisation de cellules, tissus ou organes.

**27.** Utilisation selon la revendication 24, 25 ou 26, **caractérisée en ce que** les cellules cibles sont des cellules cancéreuses.

**28.** Utilisation selon l'une des revendications 24 à 27, **caractérisée en ce que** les rayons sont appliqués en utilisant un système de radiothérapie ou de radiographie.

**Claims**

**1.** Biocompatible composite particle or nanoparticle aggregate, which can generate free radicals or heat when excited by X rays, comprising :

- a nucleus comprising a first inorganic compound absorbing X rays and emitting UV-visible energy, and a second, inorganic or organic compound, absorbing UV-visible energy and producing free radicals on contact with water or oxygen, and
- optionally, a biocompatible coating.

**2.** Particle or nanoparticle aggregate according to claim 1, **characterized in that** it has a size comprised between 4 and 250 nm, preferably between 4 and 100 nm, and more preferably between 4 and 50 nm.

**3.** Particle or nanoparticle aggregate according to one of claims 1 to 2, **characterized in that** the first inorganic compound is in the form of an oxide, hydroxide, oxysulfide or salt, doped with a rare earth element, preferably at a cations concentration less than 15% in solid solution.

**4.** Particle or nanoparticle aggregate according to one of claims 1 to 3, **characterized in that** the first inorganic compound is selected from $Y_2O_3$, $(Y,Gd)_2O_3$, $CaWO_4$, $GdO_2S$, $LaOBr$, $YTaO_3$, $BaFCl$, $Gd_2O_2S$, $Gd_3Ga_5O_{12}$, $HfGeO_4$, $Rb_3Lu(PO_4)_2$ and $Cs_3Lu(PO_4)_2$, doped with a rare earth element selected from Gd, Eu, Tb, Er, Nb, Pr and Ce.

**5.** Particle or nanoparticle aggregate according to claim 4, **characterized in that** the first inorganic compound is selected from $Y_2O_3$ doped with Gd, Eu or Tb.

**6.** Particle or nanoparticle aggregate according to claim 4, **characterized in that** the first inorganic compound is $HfGeO_4$ in mixed solution with Zr.

7. Particle or nanoparticle aggregate according to one of claims 1 to 6, **characterized in that** the second compound is an inorganic compound selected from semiconductor compounds, preferably Ti02, ZnO, CdS, CdSe, CdTe, MnTe and mixed solutions, optionally doped.

8. Particle or nanoparticle aggregate according to anyone of the preceding claims, **characterized in that** the first inorganic compound forms the core of the nucleus, and **in that** the second compound forms a layer or nanoparticles at the surface of the core.

9. Particle or nanoparticle aggregate according to anyone of the preceding claims, **characterized in that** the two inorganic compounds of the nucleus are arranged in multiple successive layers.

10. Particle or nanoparticle aggregate according to claim 9, **characterized in that** the core of the nucleus formed by the first inorganic compound has a size comprised between approximately 5 and 50 nm, and **in that** the layer formed by the second compound at the surface of the core has a thickness comprised between approximately 1 and 30 nm.

11. Particle or nanoparticle aggregate according to anyone of claims 1 to 8, **characterized in that** the two compounds of the nucleus are present in the form of a nanoparticle mixture.

12. Particle or nanoparticle aggregate according to anyone of the preceding claims, **characterized in that** the ratio of the amount or concentration of the first compound to the second compound is comprised between 0.2 and 5.

13. Particle or nanoparticle aggregate according to anyone of the preceding claims, **characterized in that** it further comprises a surface component enabling specific targeting of biological cells or tissues.

14. Particle or nanoparticle aggregate according to anyone of the preceding claims, **characterized in that** it includes a coating allowing the diffusion of small molecules and free radicals.

15. Particle or nanoparticle aggregate according to claim 14, **characterized in that** the coating is composed of a porous amorphous or crystalline structure, preferably it comprises a compound selected from silice, alumine, PEG and Dextran.

16. Particle or nanoparticle aggregate according to one of claims 13 to 15, **characterized in that** the surface component allowing specific targeting of biological cells or tissues is bound to the coating.

17. Particle or nanoparticle aggregate according to one of claims 13 to 16, **characterized in that** the surface targeting component is a biological or chemical structure displaying an affinity for molecules present in the human or animal body, such as a peptide, polypeptide, nucleotide, polynucleotide, an hormone or a vitamin.

18. Particle or nanoparticle aggregate, **characterized in that** it comprises a core comprising $Y_2O_3$:Gd covered with a layer of $TiO_2$, and a $SiO_2$-based coating

19. Particle or nanoparticle aggregate, **characterized in that** it comprises a nucleus comprising microparticles of $Y_2O_3$: Tb and of $TiO_2$, and a Dextran-based coating.

20. Particle or nanoparticle aggregate according to anyone of the preceding claims, **characterized in that** it is essentially spherical in shape.

21. Method of production of particles or nanoparticle aggregates according to anyone of claims 1 to 20, comprising:

    - mixing two compounds such as defined in claim 1 to 20 in order to form a particle or an aggregate and, optionally
    - coating the particle or aggregate.

22. Method according to claim 21, **characterized in that** it comprises:

    - preparing the core of the particle comprising the first inorganic compound,
    - covering the core thus formed with a layer comprising the second compound and, in a preferred manner,
    - coating the particle or aggregate thus obtained with a porous material.

23. Pharmaceutical or diagnostic composition comprising particles or nanoparticle aggregates according to anyone of claims 1 to 20.

24. Use of particles or nanoparticle aggregates according to anyone of claims 1 to 20, or a composition according to claim 23, combined with X rays, to prepare a medicament intended to destroy targets cells.

25. Use of particles or nanoparticle aggregates according to anyone of claims 1 to 20, or a composition according to claim 23, combined with UV rays, to prepare a medicament intended to destroy superficial target cells or target cells from cavities.

26. Use of particles or nanoparticle aggregates according to anyone of claims 1 to 20, or a composition according to claim 23, combined with X rays or UV rays, to prepare an agent intended to detect or visualize cells, tissues or organs.

27. Use according to claim 24, 25, or 26, **characterized in that** target cells are cancer cells.

28. Use according to one of claims 24 to 27, **characterized in that** rays are applied by using a system of radiotherapy or radiography.

**Patentansprüche**

1. Partikel oder Nanopartikelaggregat, biokompatibler Zusammensetzung, das in der Lage ist, freie Radikale oder Wärme bei Anregung mit Röntgenstrahlen zu erzeugen, umfassend:

   - einen Kern, umfassend eine erste anorganische Verbindung, die die Röntgenstrahlen absorbiert und die Energie im sichtbaren UV emittiert, und eine zweite anorganische oder organische Verbindung, die die Energie des sichtbaren UV absorbiert und freie Radikale bei Kontakt mit Wasser oder Sauerstoff bildet, und
   - gegebenenfalls eine biokompatible Ummantelung.

2. Partikel oder Nanopartikelaggregat gemäß Anspruch 1, **dadurch gekennzeichnet, dass** seine Größe zwischen 4 und 250 nm, bevorzugt zwischen 4 und 100 nm, noch bevorzugter zwischen 4 und 50 nm liegt.

3. Partikel oder Nanopartikelaggregat gemäß einem der Ansprüche 1 bis 2, **dadurch gekennzeichnet, dass** die erste anorganische Verbindung in Form eines Oxids, Hydroxids, Oxysulfids oder Salzes vorliegt und mit einer Seltenen Erde, vorzugsweise mit einer Kationenkonzentration von weniger als 15% in fester Lösung, dotiert ist.

4. Partikel oder Nanopartikelaggregat gemäß einem der Ansprüche 1 bis 3, **dadurch gekennzeichnet, dass** die erste anorganische Verbindung aus $Y_2O_3$, $(Y,Gd)_2O_3$, $CaWO_4$, $GdO_2S$, $LaOBr$, $YTaO_3$, $BaFCl$, $Gd_2O_2S$, $Gd_3Ga_5O_{12}$, $HfGeO_4$, $Rb_3Lu(PO_4)_2$ und $Cs_3Lu(PO_4)_2$ ausgewählt ist und mit einer Seltenen Erde dotiert ist, die aus Gd, Eu, Tb, Er, Nb, Pr und Ce ausgewählt ist.

5. Partikel oder Nanopartikelaggregat gemäß Anspruch 4, **dadurch gekennzeichnet, dass** die erste anorganische Verbindung aus $Y_2O_3$ ausgewählt ist, das mit Gd, Eu oder Tb dotiert ist.

6. Partikel oder Nanopartikelaggregat gemäß Anspruch 4, **dadurch gekenntzeichnet, dass** die erste anorganische Verbindung $HfGeO_4$ in Mischlösung mit Zr ist.

7. Partikel oder Nanopartikelaggregat gemäß einem der Ansprüche 1 bis 6, **dadurch gekennzeichnet, dass** die zweite Verbindung eine anorganische Verbindung ist, die aus den gegebenenfalls dotierten Halbleiter-Verbindungen, vorzugsweise $TiO_2$, $ZnO$, $CdS$, $CdSe$, $CdTe$, $MnTe$ und Mischösungen, ausgewählt ist.

8. Partikel oder Nanopartikelaggregat gemäß einem der vorhergehenden Ansprüche, **dadurch gekennzeichnet, dass** die erste anorganische Verbindung das Kerninnere bildet und dass die zweite Verbindung in Form einer Schicht oder in Form von Nanopartikeln auf der Oberfläche des Kerninneren vorliegt.

9. Partikel oder Nanopartikelaggregat gemäß einem der vorhergehenden Ansprüche, **dadurch gekennzeichnet, dass** die beiden anorganischen Verbindungen des Kerns in mehreren aufeinanderfolgenden Schichten angeordnet sind.

**10.** Partikel oder Nanopartikelaggregat gemäß Anspruch 9, **dadurch gekennzeichnet, dass** das von der ersten anorganischen Verbindung gebildete Kerninnere eine Abmessung zwischen ungefähr 5 und 50 nm aufweist, und dass die von der zweiten Verbindung gebildete Schicht auf der Oberfläche des Kerninneren eine Dicke zwischen ungefähr 1 und 30 mm besitzt.

**11.** Partikel oder Nanopartikelaggregat gemäß einem der Ansprüche 1 bis 8, **dadurch gekennzeichnet, dass** die beiden Verbindungen des Kerns als ein Gemisch aus Nanopartikeln vorliegen.

**12.** Partikel oder Nanopartikelaggregat gemäß einem der vorhergehenden Ansprüche, **dadurch gekennzeichnet, dass** das Mengen- oder Konzentrationsverhältnis von erster Verbindung zu zweiter Verbindung zwischen 0,2 und 5 liegt.

**13.** Partikel oder Nanopartikelaggregat gemäß einem der vorhergehenden Anspruche, **dadurch gekennzeichnet, dass** es außerdem eine Oberflächenkomponente umfasst, die ein spezifisches Zielen auf Zellen oder biologische Gewebe erlaubt.

**14.** Partikel oder Nanopartikelaggregat gemäß einem der vorhergehenden Ansprüche, **dadurch gekenntzeichnet, dass** es eine Ummantelung umfasst, die die Diffusion kleiner Moleküle und freier Radikale erlaubt.

**15.** Partikel oder Nanopartikelaggregat gemäß Anspruch 14, **dadurch gekennzeichnet, dass** die Ummantelung aus einer amorphen oder kristallinen porösen Struktur besteht und vorzugsweise eine Verbindung umfasst, die aus Siliziumdioxid, Aluminiumoxid, PEG und Dextran ausgewählt ist.

**16.** Partikel oder Nanopartikelaggregat gemäß einem der Ansprüche 13 bis 15, **dadurch gekennzeichnet, dass** die Oberflächenkomponente, die ein spezifisches Zielen auf Zellen oder biologische Gewebe erlaubt, an der Ummantelung gebunden ist.

**17.** Partikel oder Nanopartikelaggregat gemäß einem der Ansprüche 13 bis 16, **dadurch gekennzeichnet, dass** die zielende Oberflächenkomponente eine biologische oder chemische Struktur ist, die eine Affinität für Moleküle aufweist, die im menschlichen oder tierischen Körper vorhanden sind, wie ein Peptid, Polypeptid, Nukleotid, Polynukleotid, Hormon oder Vitamin.

**18.** Partikel oder Nanopartikelaggregat, **dadurch gekennzeichnet, dass** es ein Kerninneres, umfassend $Y_2O_3$:Gd, beschichtet mit einer $TiO_2$-Schicht und eine Ummantelung auf $SiO_2$-Basis umfasst.

**19.** Partikel oder Nanopartikelaggregat, **dadurch gekennzeichnet, dass** es einen Kern, umfassend $Y_2O_3$:Tb- und $TiO_2$-Mikropartikel, und eine Ummantelung auf Dextran-Basis umfasst.

**20.** Partikel oder Nanopartikelaggregat gemäß einem der vorhergehenden Ansprüche, **dadurch gekennzeichnet, dass** es eine im Wesentlichen sphärische Form besitzt.

**21.** Verfahren zur Herstellung von Partikeln oder Nanopartikelaggregaten gemäß einem der Ansprüche 1 bis 20, umfassend:

- das Mischen der beiden Verbindungen, wie sie in den Ansprüchen 1 bis 20 definiert sind, um ein Partikel oder Aggregat zu bilden, und gegebenenfalls
- die Ummantelung des Partikels oder Aggregats.

**22.** Verfahren gemäß Anspruch 21, **dadurch gekennzeichnet, dass** es umfasst;

- die Herstellung des Kerninneren des Partikel, umfassend die erste anorganische Verbindung,
- die Beschichtung des auf diese Weise gebildeten Kerninneren mit einer Schicht, umfassend die zweite Verbindung, und vorzugsweise
- die Ummantelung des auf diese Weise erhaltenen Partikels oder Aggregats mit einem porösen Material.

**23.** Pharmazeutische oder diagnostische Zusammensetzung, umfassend Partikel oder Nanopartikelaggregate gemäß einem der Ansprüche 1 bis 20.

**24.** Verwendung von Partikeln oder Nanopartikelaggregaten gemäß einem der Ansprüche 1 bis 20 oder einer Zusammensetzung gemäß Anspruch 23, in Kombination mit Röntgenstrahlen, für die Herstellung eines Medikaments für die Zerstörung von Zielzellen.

25. Verwendung von Partikeln oder Nanopartikelaggregaten gemäß einem der Ansprüche 1 bis 20 oder einer Zusammensetzung gemäß Anspruch 23, in Kombination mit UV-Strahlen, für die Herstellung eines Medikaments für die Zerstörung von superfiziellen Zielzellen oder von Zielzellen in Hohlräumen.

26. Verwendung von Partikeln oder Nanopartikelaggregaten gemäß einem der Ansprüche 1 bis 20 oder einer Zusammensetzung gemäß Anspruch 23, in Kombination mit Röntgen- oder UV-Strahlen, für die Herstellung eines Agens für die Detektion oder für die Visualisierung von Zellen, Geweben oder Organen.

27. Verwendung gemäß Anspruch 24, 25 oder 26, **dadurch gekennzeichnet, dass** die Zielzellen Krebszellen sind.

28. Verwendung gemäß einem der Ansprüche 24 bis 27, **dadurch gekennzeichnet, dass** die Strahlen unter Verwendung einer Radiotherapie- oder Radiographievorrichtung appliziert werden.

**Segment de liaison**

**Enrobage**

Fig 1A

**Premier composé inorganique**

**Deuxième composé**

Fig 1B

**ciblage**

Fig 1C

Fig 1D

**Figure 1**

**Absorption d'énergie**

Rayon X

UV

$O2^2$, $H2O^1$

$O°$, $OH°$

Chaleur

**Figure 2**

**Figure 3**

**Figure 4**

Figure 5

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 1162626 A

- US 6514481 B

**Littérature non-brevet citée dans la description**

- **MCCAUGHAN, J.S. JR.** Photodynamic Therapy: A Review. *Drugs and Aging.,* 1999, vol. 15, 49-68
- **SHIBATA et al.** *Bioscience Biotechnology and Biochemistry,* 1998, vol. 62, 2306-2311
- **NELSON et al.** Nanocrystalline Y2O3:Eu Phosphors Prepared by Alkalide Reduction. *Chem. Mater.,* 2003, vol. 15, 688-693

- **LIU et al.** Preparation and characterization of amino-silane modified superparamagnetic silica nanospheres. *Journal of Magnetism and Magnetic Materials,* 2004, vol. 270, 1-6
- **LÉVY et al.** *Chem. Mater.,* 2002, vol. 14 (9), 3715